# EUROPEAN PATENT APPLICATION

(11) **EP 1 634 562 A1**
(43) Date of publication of application: **15.03.2006**
(21) Application number: 04019733.7
(22) Date of filing: 19.08.2004
(51) Int. Cl.: A61K 6/083

(54) **Dental filler particles and method for their preparation and use**

(71) Applicant: DENTSPLY DETREY GmbH, 78467 Konstanz (DE)
(72) Inventor: Blackwell, Gordon B., 78465 Konstanz (DE); Facher, Andreas, 78467 Konstanz (DE); Grasmüller, Martin, 78467 Konstanz (DE)
(74) Representative: Hartz, Nikolai

(57) **Abstract**

A method for producing dental filler particles, comprising
(a) dispersing a polymerisable composite material comprising
   (a1) a polymerisable matrix,
   (a2) a solid particulate filler, and
   (a3) a polymerisation initiator,
   in a liquid medium,
(b) mixing the dispersion to reduce the particle size of the dispersed polymerisable composite material, and
(c) polymerising the polymerisable composite material to obtain dental filler particles.

## Description

### Field of the Invention

The present invention relates to a method for producing dental filler particles. Moreover, the present invention relates to dental filler particles obtainable according to the process of the invention. Furthermore, the present invention relates to a use of the dental filler particles according to the invention for the preparation of a dental composition. Finally, the present invention relates to a dental composition comprising the dental filler particles according to the invention and a polymerisable matrix.

### Background of the Invention

A composite is a structural material obtainable by combining two or more mutually insoluble substances. One substance comprises the embedding phase or matrix, and the others act as reinforcement for this phase. Dental filler particles are known as reinforcing substances in a dental composite material. Dental filler particles must fulfill strict requirements with regard to particle properties and mechanical properties of each particle when incorporated into a dental composite composition. US-A 20030032693 suggests the use of a prepolymerised filler in a dental composite. The prepolymerized filler of US-A 20030032693 is obtained by mechanical breakdown of a heat polymerized composite material. Specifically, the prepolymerized filler is prepared by mixing of fumed silica or other filler of desired size and characteristics with a polymerizable resin to prepare a paste. The paste is then heat polymerized. The resultant polymerized mass is ground to the desired particle size, for example, using a ceramic ball mill.

### Summary of the Invention

It is a problem of the present invention to provide a method for producing dental filler particles whereby particles may be obtained having superior mechanical properties and wherein the loss of expensive starting materials may be avoided.

The present invention is based on the recognition that communition of a polymerized dental composite mass to a desired particle size, for example, by using a ceramic ball mill, will result in a low yield of dental filler particles and is, therefore, uneconomical on an industrial scale.

Moreover, the present invention is based on the recognition that communition of a polymerized dental composite mass will damage the integrity of the composite particles and thereby inevitably deteriorate the mechanical properties of the dental filler particles for the following reason. During particle breakdown, a particle is subjected to a sequence of events potentially leading to particle fracture:
(1) Loading of the particle;
(2) Particle deformation;
(3) Buildup of a nonunifom stress field;
(4) Occurrence or nonoccurrence of fracturing.

Each event may affect the integrity of the polymerized matrix, the embedded filler and the interface between filler and matrix. Accordingly, the material of the filler particles obtained by comminution of a polymerized dental composite mass will have inferior mechanical properties as compared to the polymerized dental composite mass.

Moreover, the present invention is based on the recognition that communition of a polymerized dental composite mass will deteriorate the surface properties of the composite particles and thereby inevitably have an impact on the properties of the particle/matrix interface when the filler particles are incorporated in a dental composition.

Finally, the present invention is based on the recognition that dental filler particles may be obtained, which have superior mechanical properties while avoiding loss of expensive starting materials by a method for producing dental filler particles, comprising
(a) dispersing a polymerisable composite material comprising
   (a1) a polymerisable matrix,
   (a2) a solid particulate filler, and
   (a3) a polymerisation initiator,
      in a liquid medium,
(b) mixing the dispersion to reduce the particle size of the dispersed polymerisable composite material, and
(c) polymerising the polymerisable composite material to obtain dental filler particles.

### Detailed Description of the Preferred Embodiments

It has been found that composite particles having superior mechanical properties may be produced with minimum waste using a dispersion technique. In this technique, a composite material is dispersed in an uncured state in a liquid with which it is immiscible. By suitable stirring and dispersion techniques the composite may be broken down into particles with the required particle properties such as modality, average size, and particle size distribution. Specifically, by adjusting the stirring rate and the addition of suitable dispersants, the particle size may be adjusted to that required within a narrow range. After the required size and distribution has been achieved, the composite is hardened by conventional means such as heat or light and separated from the immiscible liquid part. The result is hardened composite particles with the required particle size distribution, and very little or no loss of material.

According the method of the present invention, a polymerisable composite material comprising
(a1) a polymerisable matrix,
(a2) a solid particulate filler, and
(a3) a polymerisation initiator,

is dispersed in a liquid medium.

The polymerisable matrix comprises polymerizable monomers or prepolymers. Specific examples of monomers contained in the polymerisable matrix include acrylic acid, methyl acrylate, ethyl acrylate, 2-hydroxyethyl acrylate, butyl acrylate, dodecyl acrylate, octyl acrylate, phenyl acrylate, methacrylic acid, methyl methacrylate, ethyl methacrylate, 2-hydroxyethyl methacrylate, butyl methacrylate, octyl methacrylate, acrylonitrile and acrylamide; maleic acid, monobutyl maleate, dibutyl maleate; vinyl acetate, vinyl benzoate, urethane dimethacrylate resin, bis-GMA, ethoxylated Bisphenol A dimethacrylate, diethyleneoxide dimethacrylate, triethyleneoxide dimethacrylate, tetraethyleneoxide dimethacrylate, glycerol dimethacrylate, trimethylolpropane trimethacrylate, pentaerythritol tetramethacrylate, pentaerythritol trimethacrylate, and dipentaerythritol hexaacrylate.

Additionally, the polymerisable matrix may contain additional polymerisable monomers selected from 1-alkenes, such as 1-hexene, 1-heptene; branched alkenes, such as vinylcyclohexane, 3,3-dimethyl-1-propene, 3-methyl-1-diisobutylene, 4-methyl-1-pentene; vinyl esters, such as vinyl acetate; styrene, substituted styrenes having an alkyl substituent in the side chain, e.g. alpha-methylstyrene, substituted styrenes having an alkyl substituent on the ring, such as vinyltoluene and p-methylstyrene, halogenated styrenes, such as monochlorostyrenes and dichlorostyrenes; or heterocyclic vinyl compounds, such as 2-vinylpyridine, 3-vinylpyridine, 2-methyl-5-vinylpyridine, 3-ethyl-4-vinylpyridine, 2,3-dimethyl-5-vinylpyridine, vinyl-pyrimidine, vinylpiperidine, 9-vinylcarbazole, 3-vinylcarbazole, 4-vinylcarbazole, 1-vinylimidazole, 2-methyl-1-vinylimidazole, N-vinylpyrrolidone, 2-vinyl-pyrrolidone, N-vinylpyrrolidine, 3-vinylpyrrolidine, N-vinylcaprolactam, N-vinylbutyrolactam, vinyloxolane, vinylfuran; vinyl and isoprenyl ethers; maleic acid derivatives, such as maleic anhydride, methylmaleic anhydride, maleimide, methylmaleimide; and dienes, such as divinylbenzene. The additional monomers may be employed as a mixture. The additional monomers may be used to adjust the mechanical properties of the dental filler particles as the case requires.

The amount of the polymerisable matrix in the polymerisable composite material is preferably from 10 to 40% by weight and particularly preferably from 15 to 30 % by weight.

The solid particulate filler may be an inorganic or organic filler or combinations thereof. A suitable inorganic particulate filler may include fused silica, quartz, crystalline silica, amorphous silica, soda glass beads, glass rods, ceramic oxides, particulate silicate glass, radiopaque glasses (barium and strontium glasses), and synthetic minerals. It is also possible to employ finely divided materials and powdered hydroxyl-apatite, although materials that react with silane coupling agents are preferred. Also available as a filler are colloidal or submicron silicas coated with a polymer. Suitable inorganic fillers are also La₂O₃, ZrO₂, BiPO₄ CaWO₄, BaWO₄ SrF₂, Bi₂O₃. Suitable organic fillers include polymer granulates such as polytetrafluoroethylene particles. Small amounts of pigments to allow matching of the composition to various shades of teeth can be included. The filler particles would be generally smaller than about 5 microns in diameter and preferably smaller than 3 µm, preferably in a range of from 0.1 to 1 µm. The filler is preferably contained in the composite material in an amount of 30 to 90 % by weight, more preferably from 50 to 85 % by weight. Moreover, the filler is preferably contained in the composite material in an amount of from 10 to 85 % by volume, more preferably 25 to 80 % by volume.

The polymerisation initiator is not particularly limited and may be selected from the group consisting of a photoinitiator, a thermal initiator or a combination of a photoinitiator and a thermal initiator. Preferred initiators include peroxy compounds, such as tert-butyl 2-ethyl-perhexanoate, benzoyl peroxide, dibenzolyl peroxide, dicumyl peroxide, methyl ethyl ketone peroxide, acetylacetone peroxide, dilauroyl peroxide, ketone peroxide, methyl isobutyl ketone peroxide, cyclohexanone peroxide, tert-butylperoxy benzoate, tert-butyl peroxy isopropyl carbonate, 2,5-bis(2-ethylhexanoylperoxy)-2,5-dimethyl-hexane, tert-butylperoxy 2-ethylhexanoate, tert-butyl-peroxy 3,5,5-trimethylhexanoate, 1,1-bis(tert-butylperoxy)cyclohexane, 1,1-bis(tert-butyl-peroxy)-3,3,5-tri-methylcyclohexane, cumyl hydroperoxide, tert-butyl hydroperoxide, bis(4-tert-butylcyclohexyl) peroxydicarbonate. Preferred initiators include also azo initiators well known to the person skilled in the art, for example AIBN and 1,1-azobis-cyclohexanecarbonitrile. Use of a suitable redox system is also possible.

The liquid medium is a medium wherein the constituents of the polymerisable composite material are insoluble. Preferably, the medium is an aqueous medium, preferably water. Preferably, the dispersing medium further comprises an emulsifying agent.

As used herein, the term "emulsifying agent" refers to a surface-active agent that generally comprises a hydrophobic portion and a hydrophilic portion. Examples of emulsifying agents include detergents. The emulsifying agent may be anionic, nonionic, zwitterionic, or cationic, depending on whether they comprise one or more charged group. Anionic emulsifying agents, contain a negatively charged group and have a net negative charge. Nonionic surfactants contain non-charged polar groups and have no charge. Examples of neutral emulsifiers are ethoxylated fatty alcohols, ethoxylated fatty acids and ethoxylated phenols and fatty acid esters of polyhydric alcohols, such as pentaerythritol or sorbitol. Exemplary nonionic emulsifying agent include t-octylphenoxypolyethoxyethanol (Triton X-100), polyoxyethylenesorbitan monolaurate (Tween 20), polyoxyethylenesorbitan monolaurate (Tween 21), polyoxyethylenesorbitan monopalmitate (Tween 40), polyoxyethylenesorbitan monostearate (Tween 60), polyoxyethylenesorbitan monooleate (Tween 80), polyoxyethylenesorbitan monotrioleate (Tween 85), (octylphenoxy)polyethoxyethanol, triethyleneglycol monolauryl ether, and sorbitan monolaurate (Span 20). A zwitterionic emulsifying agent contains both a positively charged group and a negatively charged group, and has no net charge such as lecithin. A cationic emulsifying agent has a positively charged group under the conditions examined. Cationic emulsifying agent may contain quaternary amines or tertiary amines. Exemplary quaternary amine emulsifying agent include cetylpyridinium chloride, cetyltrimethylammonium bromide, cetyltrimethylammonium chloride, dodecyltrimethylammonium bromide, dodecyltrimethylammonium chloride, octyl trimethyl ammonium bromide, tetradecyltrimethylammonium bromide, tetradecyltrimethylammonium chloride, dodecylethyidimethylammonium bromide, decyltrimethylammonium bromide, dodecyltriphenylphosphonium bromide, octadecylyl trimethyl ammonium bromide, stearoalkonium chloride, olealkonium chloride, cetrimonium chloride, alkyl trimethyl ammonium methosulfate, palmitamidopropyl trimethyl chloride, quaternium 84 , and wheat lipid epoxide. Exemplary ternary amine emulsifying agents include octyldimethylamine, decyidimethylamine, dodecyidimethylamine, tetradecyldimethylamine, hexadecyidimethylamine, octyldecyldimethylamine, octyidecylmethylamine, didecylmethylamine, dodecylmethylamine, triacetylammonium chloride, cetrimonium chloride, and alkyl dimethyl benzyl ammonium chloride. Additional classes of cationic surfactants include, but are not limited to, phosphonium, imidzoline, and ethylated amine groups. Examples of anionic emulsifiers are alkali metal salts of higher fatty acids having from 8 to 30 carbon atoms, such as stearic or oleic acid, alkali metal salts of sulphonic acids having from 8 to 30 carbon atoms, in particular sodium salts of alkyl- or arylalkylsulphonic acids, alkali metal salts of half-esters of phthalic acid, and alkali metal salts of resin acids, such as abietic acid. Certain polymers such as polyacrylic acid, polyvinyl alcohol, partially hydrolyzed polyvinyl acetate, polyvinyl pyrrolidone, methyl cellulose and hydroxyethyl cellulose may also be used.

The amount of the emulsifiers is preferably in the range from 0 to 10 % by weight, particularly preferably from 0.3 to 8% by weight, based on the weight of the dispersant. Solid substances capable of hindering coagulation of individual particles may also be used. Examples are calcium carbonate, talcum powder, hydroxyapatite, glass, or silica particles.

Usual additives and auxiliaries may also be added to the mixture prior to, during or after formation of the dispersion. These include particularly substances which give the particles particular properties, for example polymers, dyes and pigments. Use may also be made of complexing agents, such as EDTA or Trilon A, and compounds which inhibit the formation of tank deposit, such as polyethylene glycol. Polymerisation of the matrix may be carried out subsequent to mixing of the dispersion to reduce the particle size, when the dispersed polymerisable composite material is not under agitation or during mixing agitation. Moreover, it is possible to begin polymerisation when the mixture is still under agitation and finish polymerisation after mixing the dispersion.

The polymerisable composite material may be dispersed in the liquid medium by any suitable method such as mixing, stirring or blending. It is also possible to combine the step of dispersing and mixing so that the polymerisable composite material is introduced into the a device for dispersing and mixing. According to the method of the present invention, the dispersion is mixed to reduce the particle size of the dispersed polymerisable composite material. The average particle size of the disersed polymerisable composite material in step (b) is preferably reduced below 100 µm. The mixing is conducted by using a high shear force.

The dispersion may be prepared using any process suitable for this purpose. Common dispersers such as an ULTRA-TURRAX of IKA, Germany, a pressure homogenizer, e.g. from Gaulin, Lubeck, Germany, or a DISPERSMAT from VMA-Getzmann, Reichshof, Germany may be mentioned. Other devices with a rotor-stator system, such as the DISPAX from Janke&Kunkel, Germany; CAVITRON homogenizers from V. Hagen & Funke, Germany; or homogenizers from Kotthoff, Germany and homogenizers from Doee Oliver, Germany may also be used.

The ULTRA TURRAX devices are usually operated at rotation rates of 10000 to 50000 rpm, preferably from 15000 to 30000 rpm. The high shear forces required to form the dispersion may also be achieved by exposure to ultrasound, passing the mixture to be dispersed through small-diameter nozzles or through a narrow gap under high pressure, or with the aid of colloid mills.

The dispersion of the monomers and the other constituents of the reaction mixture preferably takes place at temperatures in the range from 0 to 100°C, preferably in the range from 20 to 70°C.

The dispersion time depends on the particle properties to be achieved and may be within a wide range. The dispersion can generally be prepared within 1 minute to 120 minutes.

According to the method of the present invention a disperse phase composed of a polymerisable composite is supplied into a shear force generating field within a mixing apparatus, where the particle size of the particles is reduced by shear force, whereby a dispersion of polymerizable particles of a desired size is produced. The particles are broken down by turbulent energy due to the agitation of the reaction solution or by the shear force of agitating blades. On the other hand, the particles may coalesce upon mutual contact. The final size of particles is determined by the balance between these processes of breakdown and coalescence. Since the particles are broken down in an uncured state, the events of loading of the particle, particle deformation, buildup of a nonunifom stress field, occurrence or nonoccurrence of fracturing will affect the soft uncured particle. Accordingly, the integrity of the final cured particle will not be affected by the comminution process. Moreover, the amount of particles having an undesired small particle size is efficiently reduced by the present method. Such fine particles are the fraction of the obtained particles which is below the desired particle size range. The lower limit of the desired particle size range has an influence on the maximum filler load in a dental composition. If the portion of the fine filler particles is high, then the maximum filler load in a dental composition will be reduced. According to conventional methods of comminution, such fine particles are obtained in large proportions. Due to the undesired effect of fine particles on the maximum filler load in a dental composition, the amount of the fine filler particles must be reduced. Based on an industrial production process for the preparation of dental filler particle based on milling, wherein the lower limit of the desired particle size range is defined as 3.6 µm, it was found that conventional comminution produced about 40 % of fine particles (Comparative Example 13) after a required milling time of sixteen hours. However, the method of the invention typically produces only about 20 % of fine particles outside the desired size range in less than two hours.

The shear force created by blades in the dispersing apparatus is a predominant factor in controlling the size of the particles. The size of particles formed by breakdown under the impact of blades depends on the state of particles before the breakdown, the intensity of shear force and the number of shear cycles.

Coalescence of particles occurs as a result of mutual contact of particles. Generally, the smaller the size of particles, the greater the surface energy per unit volume and the stabler the particles that are available. Further, the factor that contributes to a broader size distribution is the presence of both large and small particles in the same system. Small particles tend to be absorbed by large particles with which they collide. However, in order to make particles sufficiently small to have an adequately stable interfacial energy, a correspondingly large energy must be supplied, so it is effective to divide the particles as they are concentrated in a small shear region. In addition, it is preferable to provide conditions for regular breakdown in such a way that shear force will be applied uniformly to all the particles present.

According to the method of the present invention, the polymerisable composite material is polymerized to obtain dental filler particles. Preferably, the dental filler particles are obtained substantially in a narrow particle size range of from 3 to 100 µm. The polymerization may be carried out at atmospheric pressure, subatmospheric pressure or superatmospheric pressure. The polymerisation of the polymerisable composite material in the liquid medium may be carried out by heat and/or radiation.

The present invention also relates to dental filler particles obtainable according to the method of the invention. The dental filler particles may be advantageously used for the preparation of a dental composition. A dental composition typically comprises the dental filler particles and a polymerisable matrix. In a preferred embodiment, the polymerisable matrix is the dental composite material comprising the
(a1) polymerisable matrix and
(a2) solid filler.

The present invention will now be further illustrated by the following examples.

### EXAMPLES

### Example 1: Preparation of a first composite material

A resin mixture was first prepared by blending together UDMA resin 31.2 parts, TCB resin 21.44 parts, trimethylolpropanetrimethacrylate 6.82 parts, triethyleneglyeoldimethacrylate 6.82 parts, Plex 68780 (supplied by Röhm) 31.2 parts. To this mixture was added photoinitiators and inhibitors camphor quinone 0.31 parts, ethyldimethylamino benzoate 0.9 parts, and butylhydroxytoluene 0.81 parts, and the mixture was stirred in yellow light at 40 to 50°C until it was homogenous. This resin mixture was used to prepare a composite paste by blending 23.5 parts of the resin with 71 parts of glass powder with a mean particle size of 0.8 microns, 5 parts of strontium fluoride and 0.5 parts of aerosol in a planetary mixer. Since the resin mixture hardens under irradiation with light around 470 nm wavelength the mixing operation was carried out under yellow light with a reduced content of radiation at 490 nm.

### Example 2: Preparation of fine composite particles

A solution was prepared containing 10g Tween 85 in 1000 ml water. The blade of an Ultra TURRAX model T45 was immersed in the detergent solution and the Ultra TURRAX was started at a speed of 24000 rpm. A 5 g portion of the above paste was added to the mixture and this was stirred for 2 minutes at 24000 rpm. Further portions of 5.3 g, 10.1 g, and 10 g were then added at 2 minute intervals, stirring all the time at 24000 rpm. Due to the energy input of the stirrer the temperature of the mixture rose from 23°C to about 50°C. The mixture was stirred at high speed for a further 35 minutes after which the paste was hardened by exposing it to a 50 watt halogen lamp at close range for 30 minutes. A fine particulate solid was obtained, which was separated from the aqueous part by filtration.

### Example 3: Preparation of fine composite particles using less surfactant

A solution was prepared containing 5 g of Tween 85 in 1000 ml of water. The Tween was thoroughly dispersed by stirring with an Ultra TURRAX revolving at 24000 rpm. A 10 g portion of the paste from example 1 was then added to the stirred Tween solution and this was dispersed for 4 minutes at 24000 rpm. A further 10 g of paste was added and this was also dispersed for 4 minutes at 24000 rpm. Further 10 g lots of paste were then added, each being dispersed for two minutes at 24000 rpm before addition of the next portion. Finally the dispersed composite paste was hardened by radiation with light from a 50 watt halogen lamp at close range, for 5 minutes. The resultant fine powder was separated by filtration, washed with water, and allowed to dry.

### Example 4: Preparation of fine composite particles with longer irradiation

The experiment of example 3 was repeated except that each addition of paste was dispersed for 2 minutes at 24000 rpm. After a further dispersion time of 20 minutes, the pastes was hardened by irradiation with a 50 watt halogen lamp at close range for one and a half hours. The resultant fine powder was separated by filtration and dried at 85°C.

### Example 5: Preparation of a composite material with thermal initiators

A resin mixture was first prepared by blending together UDMA resin 31.88 parts, TCB resin 21.90 parts, trimethylolpropanetrimethacrylate 6.97 parts, triethyleneglycoldimethacrylate 6.87 parts, Plex 68780 (supplied by Röhm) 31.86 parts. 2-hydroxy-4-methoxy-benzophenone 0.24 parts at 40 to 50°C until it was homogenous. To this mixture was added thermal initiator Interox TBPEH 0.2 parts (supplied by DEGUSSA) and the mixture was stirred until it was homogenous. This resin mixture was used to prepare a composite paste by blending 27.0 parts of the resin with 73 parts of glass powder with a mean particle size of 0.8 microns in a planetary mixer. Since the resin mixture hardens thermally it was taken care that temperature did not exceed 40°C and the product was stored in the fridge.

### Example 6: Preparation of fine composite particles with simultaneous particle formation and particle hardening

A solution was prepared containing 50 g Tween 85 in 1000 ml water. The blade of an Ultra TURRAX model T45 was immersed in the detergent solution and the Ultra TURRAX was started at a speed of 10000 rpm. The solution was heated with an additional heating plate to about 80°C, 40 g of the above paste were added in portions of about 5 g within 5 minutes. Stirring at 10000 rpm was continued for an hour. A fine particulate solid was obtained, which was separated from the aqueous part by filtration. After drying a greyish powder was obtained. Particle size was determined by LALLS measurement using Mie theory (Refractive index 1,48). Bimodal size distribution with main peak from 4 to 30 microns and small peak around 1 micron was obtained.

### Example 7: Preparation of fine composite particles with subsequent particle formation and particle hardening

A solution was prepared containing 60 g Tween 85 in 1000 ml water. The blade of an Ultra TURRAX model T45 was immersed in the detergent solution and the Ultra TURRAX was started at a speed of 10000 rpm. 50 g of the above composite material were added in portions of about 5 g within 5 minutes. After additional 5 minutes of Ultra TURRAX agitation the resulting fine milk-like dispersion was transferred to a stirred vessel. There the dispersion was heated up to 87°C under continuous gentle stirring. Some larger particles could not be kept in dispersion and stuck to the walls of the vessel. After 90 minutes the hardened composite particles were filtered off and dried. After drying a white powder was obtained. Particle size was determined by LALLS measurement using Mie theory (Refractive index 1,48). Bimodal size distribution with small peak from 4 to 30 microns and main peak around 1 micron was obtained.

### Example 8: Preparation of a composite material with high viscosity and containing thermal initiators

A resin mixture was first prepared by blending together UDMA resin 31 .86 parts, TCB resin 21.90 parts, trimethylolpropanetrimethacrylate 6.97 parts, triethyleneglycoldimethacrylate 6.97 parts, Plex 68780 (supplied by Röhm) 31.86 parts, 2-hydroxy-4-methoxy-benzophenone 0.24 parts at 40 to 50°C until it was homogenous. To this mixture was added thermal initiator Interox TBPEH 0.2 parts and the mixture was stirred until it was homogenous. This resin mixture was used to prepare a composite paste by blending 21.0 parts of the resin with 79.0 parts of glass powder with a mean particle size of 0.8 microns in a planetary mixer. Since the resin mixture hardens thermally it was taken care that temperature did not exceed 40°C and the product was stored in the fridge.

### Example 9: Preparation of fine composite particles with subsequent particle formation and particle hardening from composite material with high viscosity

A solution was prepared containing 60 g Tween 85 in 1000 ml water. The blade of an Ultra TURRAX model T45 was immersed in the detergent solution and the Ultra TURRAX was started at a speed of 10000 rpm. The solution was heated with an additional heating plate to about 80°C. Fifty grams of the above composite material were added in portions of about 5 g within 5 minutes. After additional 5 minutes of Ultra TURRAX high shear mixing the resulting fine milk-like dispersion was transferred to a stirred vessel. There the dispersion was heated up to 87°C under continuous gentle stirring. After 90 minutes the hardened composite particles were filtered off and dried. After drying a white powder was obtained. Particle size was determined by LALLS measurement using Mie theory (Refractive index 1,48). Broad size distribution with main peak around 1 micron and steady tailing up to 80 micron was obtained.

### Example 10: Comparative Example: Preparation of a thermally hardenable material without fillers

A hardenable resin was prepared by blending together dodecanedioldimethacrylate 97.0 parts and benzoyl peroxide 3.0 parts until it was homogenous. Since the resin mixture hardens thermally it was taken care that temperature did not exceed 40°C and the product was stored in the fridge.

### Example 11: Preparation of fine particles from comparative material without fillers

The blade of an Ultra TURRAX model T45 was immersed in water and the Ultra TURRAX was started at a speed of 10000 rpm. This continuous phase was heated with an additional heating plate to about 70°C. 50 g of the above composite material were added in portions of about 5 g within 5 minutes. After 30 minutes of Ultra TURRAX agitation the dispersed liquid drops were hardened.

Particle size was determined by LALLS measurement using Mie theory (Refractive Index 1,48). A trimodal size distribution was obtained with main peak around 150 micron, second peak at 10 and third at 1 micron.

### Example 12 (COMPARATIVE): Preparation of fine glass filler particles by milling

A porcelain ball mill with an internal diameter of 10 cm and height of 10 cm was charged with 650 g of zirconium oxide cylinders approximately 1 cm in diameter and 1 cm long. Ninety grams of glass frit with a particle size between about 0.1 µm and 3 mm was added and the mill was sealed. The mill was set to revolve on rollers at 100 rpm for 32 hours, after which the glass and zirconium oxide cylinders were separated using a coarse metal grid. A few remaining large glass particles were removed by passing the material through a 180 µm sieve. The milled glass was found to have a mean size of 5.6 µm and a range from 0.5 to 76 µm.

### Example 13 (COMPARATIVE): Preparation of fine composite particles by milling

A porcelain ball mill with an internal diameter of 10 cm and height of 10 cm was charged with 650 g of aluminum oxide cylinders approximately 1 cm in diameter and 1 cm long. Ninety grams of hardened composite material with a particle size between about 0.1 µm and 3 mm was added and the mill was sealed. The mill was set to revolve an rollers at 100 rpm for 32 hours, after which the composite and aluminum oxide cylinders were separated using a coarse metal grid. Visual inspection of the milled composite showed that there was still a large amount of large particulate matter present, and that milling was very slow. The composite was replaced in the mill together with enough ceramic spheres 2.5 cm in diameter that these were just covered by the composite material. The mill was turned for a further 16 hours, after which the ceramic spheres were separated. The particle size of the milled composite was measured and found to have an overall mean size of 13.3 µm and a range from 0.1 µm to 220 µm.

**Table 1**

| Exp. Conditions | example 2 | example 6 | example 7 | example 9 | example 11 | example 12 | example 13 |
|---|---|---|---|---|---|---|---|
| material | composite | composite | composite | composite | composite | glass frit | composite |
| method | dispersion | dispersion | dispersion | dispersion | dispersion | dry mill | dry mill |
| initiator | light/ CO/DMABE | thermal TBPEH | thermal TBPEH | thermal TBPEH | thermal TBPEH | - | thermal TBPEH |
| % filler In paste | 76.5 | 73.0 | 73.0 | 79.0 | 0.0 | - | 73.0 |
| paste consistency | normal | normal | normal | crumbly | pure resin | - | normal |
| % Tween 85 | 1.0 | 5.0 | 6.0 | 6.0 | 0.0 | - | - |
| Temp. °C | 50°C | 100 | 23 | 80 | 70 | - | - |
| Dispersion time minutes | 70 | 60 | 10 | 10 | 30 | - | - |
| distribution type | | bimodal | bimodal | monomodal | trimodal | monomodal | bimodal |
| size range of main peak | | 4 to 30 µ | 4 to 30 µ | 1 to 80 µ | 10 to 150 µ | 0.5 to 76 µ | 1 to 220 µ |
| mean partide size of minor peak | | -1 µ | -1 µ | - | -10 µ and -1 µ | | -1 µ |

## Claims

1. A method for producing dental filler particles, comprising
(a) dispersing a polymerisable composite material comprising
(a1) a polymerisable matrix,
(a2) a solid particulate filler, and
(a3) a polymerisation initiator,
in a liquid medium,
(b) mixing the dispersion to reduce the particle size of the dispersed polymerisable composite material, and
(c) polymerising the polymerisable composite material to obtain dental filler particles.

2. The method according to claim 1, wherein the average particle size of the disersed polymerisable composite material in step (b) is reduced below 100 µm.

3. The method according to claim 1 or 2, wherein dental filler particles are obtained substantially in a narrow particle size range of from 3 to 100 µm.

4. The method according to any one of the preceding claims wherein the dispersing medium is an aqueous medium.

5. The method according to any one of the preceding claims wherein the dispersing medium further comprises an emulsifying agent.

6. The method according to any one of the preceding claims wherein the polymerisable matrix comprises a monomer selected from the group consisting of acrylic acid, methyl acrylate, ethyl acrylate, 2-hydroxyethyl acrylate, butyl acrylate, dodecyl acrylate, octyl acrylate, phenyl acrylate, methacrylic acids, methyl methacrylate, ethyl methacrylate, 2-hydroxyethyl methacrylate, butyl methacrylate, octyl methacrylate, acrylonitrile and acrylamide; maleic acid, monobutyl maleate, dibutyl maleate; vinyl acetate, vinyl benzoate, and urethane dimethacrylate resin, bis-GMA, ethoxylated Bisphenol A dimethacrylate, diethyleneoxide dimethacrylate, triethyleneoxide dimethacrylate, tetraethyleneoxide dimethacrylate, glycerol dimethacrylate, glicerine dimethacrylate, trimethylolpropane trimethacrylate, pentaerythritol tetramethacrylate, pentaerythritol trimethacrylate, and dipentaerythritol hexaacrylate.

7. The method according to any one of the preceding claims wherein the solid particulate filler is selected from the group consisting of an inorganic filler or an organic filler.

8. The method according to claim 7 wherein the filler is contained in the composite material in an amount of from 30 to 90 % by weight.

9. The method according to claim 7 wherein the filler is contained in the composite material in an amount of from 10 to 85 % by volume.

10. The method according to any one of the preceding claims wherein the polymerisation initiator is selected from the group consisting of a photoinitiator, a thermal initiator or a combination of a photoinitiator and a thermal initiator.

11. The method according to any one of the preceding claims wherein the polymerisation initiator is selected from the group consisting of tert-butyl 2-ethyl-perhexanoate, benzoyl peroxide, dibenzolyl peroxide, dicumyl peroxide, methyl ethyl ketone peroxide, acetylacetone peroxide, dilauroyl peroxide, ketone peroxide, methyl isobutyl ketone peroxide, cyclohexanone peroxide, tert-butylperoxy benzoate, tert-butyl peroxy isopropyl carbonate, 2,5-bis(2-ethylhexanoylperoxy)-2,5-dimethyl-hexane, tert-butylperoxy 2-ethylhexanoate, tert-butyl-peroxy 3,5,5-trimethylhexanoate, 1,1-bis(tert-butylperoxy)cyclohexane, 1,1-bis(tert-butyl-peroxy)-3,3,5-tri-methylcyclohexane, cumyl hydro-peroxide, tert-butyl hydroperoxide, bis(4-tert-butylcyclohexyl) peroxydicarbonate, AIBN and 1,1-azobis-cyclohexanecarbonitrile.

12. The method according to any one of the preceding claims wherein the high shear mixing is carried out by an ULTRA TURRAX mixer.

13. The method according to any one of the preceding claims wherein the high shear mixing is carried out by an ULTRA TURRAX mixer at a speed of from 10000 to 50000 rpm.

14. The method according to any one of the preceding claims wherein polymerising the polymerisable composite material in the liquid medium is carried out by heat and/or radiation.

15. Dental filler particles obtainable according to the method of any one of the preceding claims.

16. Use of the dental filler particles according to claim 15 for the preparation of a dental composition.

17. Dental composition comprising the dental filler particles according to claim 15 and a polymerisable matrix.

18. The dental composition according to claim 17, wherein the polymerisable matrix is the dental composite material comprising the
(a1) polymerisable matrix and
(a2) solid filler.
